# DEMANDE DE BREVET EUROPEEN

(11) **EP 1 281 386 A1**
(43) Date de publication de la demande: **05.02.2003**
(21) Numéro de dépôt: 02023181.7
(22) Date de dépôt: 31.10.1996
(51) Int. Cl.: A61K 7/06, A61K 7/48, A61K 7/00, A61K 7/32, A61K 7/032, A61K 7/027

(54) **Composition cosmétique solide et utilisations**

(30) Priorité: 06.11.1995 FR 9513094
(62) Demande divisionnaire de: 96937383.6
(71) Demandeur: L'OREAL, 75008 Paris (FR)
(72) Inventeur: Samain, Henri, 91570 Bievres (FR); Cretois, Isabelle, 78220 Viroflay (FR)
(74) Mandataire: Dodin, Catherine

(57) **Abrégé**

La présente invention concerne des compositions cosmétiques non détergentes solides qui sont non-collantes et qui ont une capacité d'absorption en eau supérieure à 50 mg. Ces compositions se présentent généralement sous forme de bâtons, de crayons ou de pains. Les compositions de l'invention peuvent constituer de nombreux produits sous forme solide appliqués pour le maquillage, pour le soin et/ou le traitement de la peau, du cuir chevelu, des cheveux ou des muqueuses, pour le coiffage et/ou la mise en forme des fibres kératiniques telles que les cheveux.

## Description

La présenté invention concerne des compositions cosmétiques non détergentes solides qui sont non-collantes et qui ont une bonne capacité d'absorption en eau ainsi que leur utilisation en cosmétique.

On connaît dans l'industrie cosmétique diverses formes de produits sous forme d'un solide notamment dans le domaine du maquillage comme les bâtons ou "sticks" de rouge à lèvres, de fond de teint ou d'ombre à paupières ; dans le domaine du soin de la peau ou des lèvres tels que les crayons réparateurs des lèvres, les bâtons ou "sticks" dépigmentants ou hydratants ; dans le domaine de l'hygiène comme les sticks déodorants. Ces compositions présentent certains inconvénients. Comme les actifs sont délivrés directement à partir de la composition solide, ces diverses compositions doivent se présenter dans un conditionnement hermétique pour ne pas se dessécher à l'air ou pour ne pas tacher. De nombreuses compositions solides sont formulées à base de cires, elles ont un caractère gras qui n'est pas apprécié par les utilisateurs.
D'autre part, certaines compositions telles que les produits de coiffage sont en général présentés sous forme fluide tel que les gels ou les mousses. Il est apparu intéressant de pouvoir disposer de produit sous forme d'un solide.

La demanderesse a découvert de manière surprenante de nouvelles compositions cosmétiques sous la forme d'un solide présentant des caractéristiques particulières.
Les compositions selon l'invention présentent l'avantage d'être hydratables en surface en contact avec de l'eau ou une surface humide au moment de l'utilisation, de permettre une bonne libération des produits cosmétiquement actifs sur la matière kératinique à traiter, de reprendre rapidement, après séchage, leur forme solide initiale sans altération et de pouvoir être réutilisées ultérieurement par simple hydratation en surface.
Les compositions selon l'invention présentent également l'avantage de pouvoir contenir des composés hydrophobes et/ou lipophiles, mis en dispersion sans l'aide de tensioactif. Les compositions selon l'invention peuvent se présenter sous forme de bâton, de crayon ou de pain et constituer en elles-mêmes de nouveaux types de produits de maquillage tels que des rouges à lèvres, des fonds de teint, des ombres à paupières ; de nouveaux types de produits de soin et/ou de conditionnement des cheveux tels que des gels durs de coiffage ; des nouveaux types de produits sous forme de bâton pour le soin du visage ou du corps.

L'invention a donc pour objet une composition cosmétique non détergente sous forme d'un solide, comprenant dans un milieu cosmétiquement acceptable, un agent gélifiant choisi dans le groupe formé par l'agar-agar, les carraghénanes, les alginates; la gomme de caroube, les gommes de guar ou leurs dérivés; la gomme arabique, la gomme karaya, la gomme adragante, la gomme de gatty ; la gomme de xanthane, les celluloses ou leurs dérivés ; les pectines ; la gélatine, les casemates ; les acides polyacryliques réticulés ; les hectorites synthétiques, les silicates d'aluminium et de magnésium, et présent à une concentration comprise entre 8% et 90% en poids par rapport au poids total de la composition, ladite composition présentant une capacité d'absorption d'eau supérieure à 50 mg et un collage à l'état non hydraté inférieur à 0,2 g.

Le terme non détergent signifie que la composition ne permet pas d'éliminer d'un milieu solide tel que par exemple la peau ou les cheveux, les salissures qui y adhèrent par leur mise en dispersion ou en solution.
Par hydratable, on entend selon l'invention une composition qui présente une capacité d'absorption d'eau selon le test décrit ci-dessous supérieure à 50 mg, de préférence supérieure à 100 mg et inférieure à 3000 mg et plus particulièrement comprise entre 200 et 1200 mg.
Par composition ne présentant pas un toucher collant, on entend selon l'invention une composition dont le collage à l'état non hydraté défini selon le test décrit ci-dessous est inférieur 0,2 g, de préférence compris entre 0 et 0,1 g.
L'état non hydraté correspond à l'état initial de la composition plus particulièrement avant l'emploi, c'est-à-dire avant la mise en contact avec de l'eau ou une surface humide.

Le collage de la composition à l'état non hydraté est déterminé par le poids de produit retiré par un papier absorbant appliqué pendant 10 secondes sur la composition solide.
La composition se trouve dans une boite de Pétri ronde de 4 cm de diamètre, la composition présente une épaisseur de 8 mm sur un diamètre de 4 cm. La surface de la composition est plane et horizontale. On applique un morceau de papier absorbant carré(Sopalin de grammage égal à 45 g/m²) de 4 cm² (2cmx2cm) que l'on a préalablement pesé (P₁). Après 10 secondes de temps de contact, on retire le papier, puis on le pèse de nouveau (P₂) et on calcule la quantité de produit retiré (P₂- P₁).

La capacité d'absorption d'eau est déterminée par le poids d'eau absorbé en 10 secondes par la composition solide.
La composition se trouve dans une boite de Pétri ronde de 4 cm de diamètre, la composition présente une épaisseur de 8 mm sur un diamètre de 4 cm. La surface de la composition est plane et horizontale. On pèse la coupelle contenant la composition, puis on verse 5 g d'eau sur la composition. Après 10 secondes de temps de contact, on évacue l'eau non absorbée. On pèse de nouveau la coupelle et on calcule la quantité d'eau absorbée.

Plus particulièrement, la composition comprend au moins un agent gélifiant dans un milieu cosmétiquement acceptable.

Le milieu cosmétiquement acceptable est de préférence aqueux, c'est-à-dire qu'il comprend soit uniquement de l'eau, soit de l'eau et un solvant tel que par exemple l'éthanol, le propylène glycol, le butylène glycol, l'isopropanol, les éthers de glycol tel que les alkyl(C₁-C₄)éther de mono, di- ou tripropylène glycol, mono, di- ou triéthylène glycol, le dipropylène glycol, le diéthylène glycol et leurs mélanges.

Bien entendu, la quantité maximale d'eau et/ou de solvants est limitée par le fait que la composition selon l'invention doit être sous la forme d'un solide et présenter les caractéristiques décrites ci-dessus.

Selon l'invention, les agents gélifiants sont de préférence hydrosolubles ou hydrophiles.

Les agents gélifiants présents dans les compositions de l'invention, sont choisis de préférence dans le groupe formé par :
- les extraits d'algues tels que l'agar-agar, les carraghénanes, les alginates ;
- les extraits de graines tels que la gomme de caroube, la gomme de guar et leurs dérivés;
- les exsudats dé plantes tels que la gomme arabique, la gomme karaya, la gomme adragante, la gomme de gatty ;
- les exsudats de micro-organismes tels que la gomme de xanthane,
- la cellulose ou ses dérivés comme la carboxyméthylcellulose, l'hydroxypropylcellulose, la méthylcellulose, l'hydroxypropylméthylcellulose ou l'hydroxyéthylcellulose ainsi que les celluloses modifiées notamment par greffage de groupement alkyle ;
- les extraits de fruits tels que les pectines ;
- les agents gélifiants d'origine animale tels que la gélatine, les casemates ;
- les polymères synthétiques gélifiants hydrosolubles tels que les acides polyacryliques réticulés tels que les "CARBOPOL" ou " PEMULEN" de la Société GOODRICH ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT.
- les mélanges des composés ci-dessus.

Les agents gélifiants sont de préférence choisis parmi :
- les extraits de graines tels que la gomme de caroube, la gomme de guar et leurs dérivés;
- les extraits d'algue tels que l'agar-agar, les carraghénanes, les alginates ;
- et leurs mélanges.

Encore plus particulièrement, on utilise selon l'invention des gommes de guar non-ioniques ou ioniques et les mélanges de gomme de caroube et de carraghénanes.

Les gommes de guar sont plus particulièrement modifiées par des groupements hydroxyalkyle en C₁-C₆.
Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation, qui correspond au nombre de molécules d'oxyde d'alkylène consommé par le nombre de fonctions hydroxyle libres présentes sur la gomme de guar, varie de préférence de 0,4 à 1,2.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société MEYHALL, sous les dénominations JAGUAR DC 293 et JAGUAR HP 105 par la société RHONE POULENC ou sous la dénomination GALACTASOL 4H4FD2 par la société AQUALON.

Les gommes de guar ioniques sont plus particulièrement des gommes de guar cationiques comportant par exemple des groupements cationiques trialkylammonium. De préférence, 2 à 30 % et encore plus préférentiellement 5 à 20 % en nombre des fonctions hydroxyle de ces gommes de guar portent des groupements cationiques trialkyammonium.
Parmi ces groupements trialkylammonium, on peut tout particulièrement citer les groupements triméthylammonium et triéthylammonium.
Encore plus préférentiellement, ces groupements représentent de 5 à 20 % en poids par rapport au poids total de la gomme de guar modifiée.
Selon l'invention, on utilise de préférence une gomme de guar modifiée par du chlorure de 2,3-époxypropyl triméthylammonium.
Ces gommes de guar modifiées par des groupements cationiques sont des produits déjà connus en eux-mêmes et sont par exemple décrits dans les brevets US 3 589 578 et US 4 0131 307. De tels produits sont par ailleurs vendus notamment sous les dénominations commerciales de JAGUAR C13 S, JAGUAR C15, JAGUAR C17 par la société MEYHALL.

Généralement, l'agent gélifiant est présent à des concentrations supérieures à 8% en poids par rapport au poids total de la composition, préférentiellement, dans des concentrations allant de 8 à 90% en poids par rapport au poids total de la composition et encore plus particulièrement entre 8 et 70% en poids.

Les compositions selon l'invention peuvent également contenir un ou plusieurs tensioactifs non ioniques, anioniques, cationiques ou amphotères, habituellement utilisés en cosmétique. La quantité d'agent tensioactif utilisée est de préférence de 0,5 à 30% par rapport au poids total de la composition. La nature et la concentration de ces tensioactifs sont choisies par l'homme du métier de façon à ne pas conférer un caractère détergent à la composition. De préférence, la composition contient moins de 4% en poids de tensioactifs détergents.

Les compositions selon l'invention peuvent également contenir un ou plusieurs polymères anioniques, cationiques, non ioniques, amphotères ou zwittérioniques. Ces polymères peuvent être présents dans des concentrations comprises entre 0,1 et 70 % en poids par rapport au poids total de la composition et de préférence entre 0,5 et 30% en poids.

Les polymères, notamment les polymères fixants sont de préférence présents dans un rapport de concentration en poids polymère / agent gélifiant compris entre 0,5 et 2.
Par polymère fixant, on entend tout polymère ayant pour fonction de fixer temporairement la forme des fibres kératiniques telles que par exemple les cheveux ou les cils.
Les polymères peuvent être dissous dans le milieu cosmétiquement acceptable ou utilisés sous forme de dispersions aqueuses de particules insolubles (latex ou pseudolatex).

Les compositions selon l'invention peuvent contenir des additifs habituellement utilisés dans les compositions cosmétiques. On peut en particulier citer les agents antioxydants ou anti-radicaux libres; les charges insolubles minérales et/ou des charges organiques, de structure lamellaire ou sphérique, les pigments ou les colorants, les silicones, les huiles et/ou les cires d'origine animale, végétale, minérale ou synthétique, les agents hydratants ou humectants tels que la glycérine et le collagène ; les filtres UV, les parfums, les agents antipelliculaires, les agents conditionneurs, les actifs déodorants.

Ces additifs peuvent être présents dans la composition finale en une quantité de 0 à 80%, de préférence de 0,5 à 50% en poids par rapport au poids total de la composition et encore plus particulièrement entre 0,5 et 15% en poids.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.

Les compositions selon l'invention peuvent être préparées par exemple par simple mélange, ou par mélange suivi de malaxage et extrusion dans une extrudeuse.
L'extrudeuse est de préférence une extrudeuse bi-vis.
L'extrudeuse pouvant être utilisée pour le procédé est choisi parmi les extrudeuses bi-vis telles que celles décrites dans la demande FR 94-00756 déposée le 25 janvier 1994.
Les matières premières sont introduites, à l'entrée de l'extrudeuse bi-vis, dans la zone d'alimentation à température ambiante, de préférence à environ 20°C, puis sont amenées dans la zone de transport à une température, de préférence, à environ 50°C, puis sont malaxées et comprimées dans diverses zones de l'extrudeuse maintenues à une température allant, préférentiellement, de 60 à 100°C ; la masse obtenue est transportée vers la sortie de l'extrudeuse et extrudée au travers d'une filière.
Pendant la phase de malaxage et de compression, l'agent gélifiant en contact avec le milieu cosmétiquement acceptable forme, après extrusion, un réseau gélifié constituant la matrice des produits finaux. La masse extrudée sort de la filière sous la forme de boudins de diamètre donné selon la filière utilisée, pouvant être ensuite découpés et mis en forme de bâton, de crayon à mine aqueuse ou de pain solide. D'autres formes peuvent bien entendu être réalisées en choisissant des filières appropriées et des dispositifs de mise en forme des produits finaux adaptés à la forme recherchée.

Les compositions solides selon l'invention peuvent se présenter sous diverses formes selon l'application choisie. Les formes les plus utilisées sont les bâtons ou "sticks", les crayons ou les pains.

Les compositions selon l'invention peuvent être des produits pour le maquillage tels que des rouges à lèvres, des fonds de teints, des ombres à paupières, des fards à joues, des anti-cernes, des mascaras. Les compositions de maquillage sont stockées à l'état non hydraté et, au moment de l'utilisation, hydratées en surface par contact avec de l'eau ou une surface humide pour délivrer les substances actives pour le maquillage puis retrouvent, après séchage, leur forme initiale sans altération, prêtes pour une autre utilisation dans les mêmes conditions.
Un autre objet de l'invention est donc un procédé de maquillage des lèvres, du visage, du contour des yeux, des joues, les cils, les sourcils ou des paupières, caractérisé par le fait que l'on utilise une composition solide telle que définie ci-dessus, que l'on mouille celle-ci en surface avec de l'eau ou une surface humide et que l'on applique ladite composition hydratée sur les lèvres, le visage, le contour des yeux, les joues, les cils, les sourcils ou les paupières.
Un autre objet de l'invention est également un procédé de maquillage des lèvres, du visage, du contour des yeux, des joues, les cils, les sourcils ou des paupières, caractérisé par le fait que l'on utilise une composition solide telle que définie ci-dessus, que l'on applique ladite composition non hydratée sur une surface humide telle que les lèvres, le visage, le contour des yeux, les joues, les cils, les sourcils ou les paupières.

Les compositions selon l'invention peuvent être également des produits pour le soin et/ou le conditionnement et/ou l'hygiène de la peau, des muqueuses, du cuir chevelu ou des cheveux. Elles sont appliquées sur les matières kératiniques, au moment de l'emploi, par simple hydratation en surface en contact avec de l'eau ou une surface humide pour délivrer les composés puis retrouvent, après séchage, leur forme initiale sans altération, prêtes pour une autre utilisation dans les mêmes conditions.
Parmi les produits de soin, de conditionnement ou d'hygiène envisageables, on peut mentionner, par exemple, en capillaire : des gels solides de coiffage en forme de pain ou de stick ; en soin de la peau : des hydratants, des amincissants en forme de stick ou de pain, des produits pour le soin des lèvres en forme de stick ou de crayon ; en hygiène : des produits pour le rasage, des déodorants, en forme de stick ou de pain.
Un autre objet de l'invention consiste en un procédé de traitement cosmétique pour le soin et/ou conditionnement et/ou l'hygiène de la peau, des cheveux, du cuir chevelu ou des muqueuses, caractérisé par le fait que l'on utilise une composition solide telle que définie ci-dessus, que l'on mouille celle-ci en surface avec de l'eau ou une surface humide et que l'on applique ladite composition partiellement hydratée sur la peau, les cheveux, les cils, les sourcils, le cuir chevelu ou les muqueuses.
Un autre objet de l'invention consiste en un procédé de traitement cosmétique pour le soin et/ou conditionnement et/ou l'hygiène de la peau, des cheveux, du cuir chevelu ou des muqueuses, caractérisé par le fait que l'on utilise une composition solide telle que définie ci-dessus, que l'on applique ladite composition non hydratée sur une surface humide telle que sur la peau, les cheveux, les cils, les sourcils, le cuir chevelu ou les muqueuses.

Selon un mode de réalisation particulier de l'invention, les compositions sont des produits de coiffage et/ou de mise en forme des fibres kératiniques telles que les cheveux ou les cils.
La présente invention a encore pour objet un procédé de coiffage et/ou de mise en forme des fibres kératiniques telles que les cheveux, caractérisé par le fait que l'on utilise une composition solide telle que définie ci-dessus, que l'on mouille celle-ci en surface avec de l'eau ou une surface humide et que l'on applique ladite composition hydratée sur lesdites fibres kératiniques.
La présente invention a encore pour objet un procédé de coiffage et/ou de mise en forme des fibres kératiniques telles que les cheveux, caractérisé par le fait que l'on utilise une composition solide telle que définie ci-dessus, que l'on applique ladite composition non hydratée sur une surface humide telle que lesdites fibres kératiniques.

Dans ce qui suit ou ce qui précède, les pourcentages sont exprimés en poids, sauf mention contraire.
Les exemples qui suivent servent à illustrer l'invention sans pour autant en limiter la portée.

### Exemple 1 Gel solide de coiffage

On a préparé un gel solide de composition suivante :
- Gomme de guar hydroxypropylée
   (Jaguar HP 60 de MEYHALL) 10,0%
- Ethanol 17,2%
- Conservateur qs
- Eau 100,0%

La gomme de guar est introduite en pluie sous agitation dans l'eau froide et l'éthanol. Après quelques minutes, on coule la composition dans un moule, puis après une attente d'environ 1 heure, on démoule la composition sous forme d'un solide.
Selon le test décrit précédemment, le collage de la composition est d'environ 60 mg.
Selon le test décrit précédemment, la capacité d'absorption en eau de la composition est d'environ 435 mg.
Le produit est hydraté par passage dans les mains mouillées, puis le produit hydraté et récupéré sur les mains est réparti sur la chevelure en passant les mains dans les cheveux mouillés. Les cheveux sont ensuite mis en forme puis séchés sous un casque.
La composition a un bon effet coiffant. Les cheveux sont brillants et présentent un toucher naturel.

### Exemple 2 (comparatif) Gel solide de coiffage

On a préparé un gel solide de composition suivante :
- Copolymère acétate de vinyle / vinylpyrrolidone
   (LUVISCOL VA 64 de BASF) 15,0 g
- Copolymère acrylique
   (SYNTHALEN K de 3V) 4,40 g
- Ethanol 17,2 g
- Conservateur qs
- Eau qsp 100,0 g

Les polymères sont introduits en pluie sous agitation dans l'eau froide et l'éthanol contenant le conservateur. Après quelques minutes, on coule la composition dans un moule, puis après une attente d'environ 1 heure, on démoule la composition sous forme d'un solide.
Selon le test décrit précédemment, le collage de la composition est d'environ 1330 mg.
Il a donc un toucher collant, il ne peut être manipulé à l'état non hydraté.
La composition a un effet coiffant mais les cheveux sont ternes et présentent un toucher collant.

### Exemple 3 Gel solide de coiffage

On a préparé un gel solide de composition suivante :
- Gomme de guar hydroxypropylée
   (Jaguar HP 60 de MEYHALL) 15,0 g
- Ethanol 17,2 g
- Conservateur qs
- Eau qsp 100,0 g

La gomme de guar est introduite en pluie sous agitation dans l'eau froide et l'éthanol contenant le conservateur. Après quelques minutes, on coule la composition dans un moule, puis après une attente d'environ 1 heure, on démoule la composition sous forme d'un solide.
Selon le test décrit précédemment, le collage de la composition est d'environ 61 mg.
Selon le test décrit précédemment, la capacité d'absorption en eau de la composition est d'environ 386 mg.
Lorsqu'il est appliqué sue les cheveux, ce gel de coiffage présente les mêmes propriétés que celui de l'exemple 1.

### Exemple 4 Gel solide de coiffage

On a préparé un gel solide de composition suivante :
- Gomme de caroube 35,0 g
- Gomme de carraghénane 35,0 g
- Conservateur qs
- Eau qsp 100,0 g

Les matières premières sont introduites à l'entrée d'une extrudeuse bi-vis à une température de 30°C. Elles sont ensuite amenées dans la zone de transport à une température de 50°C, puis sont malaxées et comprimées dans diverses zones de l'extrudeuse maintenues à environ 80-90°C. La masse ainsi malaxée et comprimée, est transportée vers la sortie de l'extrudeuse et extrudée par exemple au travers d'une filière de 3 cm de diamètre. Le temps de passage dans l'extrudeuse est d'environ 15 secondes. La vitesse de rotation des vis est de 500 tours/minute. Les boudins obtenus à la sortie de la filière sont réduits sous forme de bâtonnets de 10 cm de longueur au moyen d'un dispositif de découpe en sortie de l'extrudeuse.

Selon le test décrit précédemment, le collage de la composition est d'environ 0 mg.
Selon le test décrit précédemment, la capacité d'absorption en eau de la composition est d'environ 550 mg.
Lorsqu'il est appliqué sue les cheveux, ce gel de coiffage présente les mêmes propriétés que celui de l'exemple 1.

## Revendications

1. Composition cosmétique non détergente sous forme d'un solide, comprenant dans un milieu cosmétiquement acceptable, un agent gélifiant choisi dans le groupe formé par l'agar-agar, les carraghénanes, les alginates; la gomme de caroube, les gommes de guar ou leurs dérivés; la gomme arabique, la gomme karaya, la gomme adragante, la gomme de gatty ; la gomme de xanthane, les celluloses ou leurs dérivés ; les pectines ; la gélatine, les casemates ; les acides polyacryliques réticulés ; les hectorites synthétiques, les silicates d'aluminium et de magnésium, et présent à une concentration comprise entre 8% et 90% en poids par rapport au poids total de la composition, ladite composition présentant une capacité d'absorption d'eau supérieure à 50 mg et un collage à l'état non hydraté inférieur à 0,2 g.

2. Composition selon la revendication 1, **caractérisée par le fait que** la capacité d'absorption en eau est comprise entre 100 et 3000 mg.

3. Composition selon l'une des revendications précédentes, **caractérisée par le fait que** la capacité d'absorption en eau est comprise entre 200 et 1200 mg.

4. Composition selon l'une des revendications précédentes, **caractérisée par le fait que** le collage est compris entre 0 et 0,1 g.

5. Composition selon l'une des revendications précédentes, **caractérisée par le fait que** l'agent gélifiant est présent dans une concentration comprise entre 8% et 70% en poids par rapport au poids total de la composition.

6. Composition selon l'une des revendications précédentes, **caractérisée par le fait que** le milieu cosmétiquement acceptable est aqueux.

7. Composition selon l'une des revendications précédentes, **caractérisée par le fait qu'**elle comprend des adjuvants choisis parmi les pigments, les tensio-actifs, les antioxydants, les anti-radicaux libres, les hydratants, les silicones, les humectants, les filtres UV, les huiles et/ou des cires d'origine animale, végétale, minérale ou synthétique, les polymères anioniques, cationiques, non ioniques, amphotères ou zwittérioniques.

8. Composition selon l'une quelconque des revendications précédentes, **caractérisée par le fait qu'**elle comprend un polymère dans un rapport de concentration en poids polymère/agent gélifiant compris entre 0,5 et 2.

9. Composition selon l'une quelconque des revendications précédentes, **caractérisée par le fait qu'**elle se présente sous forme de bâton ou "stick", de crayon ou de pain.

10. Composition selon l'une quelconque des revendications précédentes, **caractérisée par le fait qu'**elle est un produit pour le maquillage.

11. Composition selon l'une quelconque des revendications 1 à 9, **caractérisée par le fait qu'**elle est un produit pour le soin et/ou le conditionnement et/ou l'hygiène de la peau, des muqueuses, du cuir chevelu ou des cheveux.

12. Composition selon l'une quelconque des revendications 1 à 9, **caractérisée par le fait qu'**elle est un produit pour le coiffage et/ou la mise en forme des fibres kératiniques telles que les cheveux ou les cils.

13. Procédé de maquillage des lèvres, du visage, des cils, des sourcils ou des paupières, **caractérisé par le fait que** l'on utilise une composition solide selon la revendication 10, que l'on mouille celle-ci en surface avec de l'eau ou une surface humide et que l'on applique ladite composition hydratée sur les lèvres, le visage, les cils, les sourcils ou les paupières.

14. Procédé de maquillage des lèvres, du visage, des cils, des sourcils ou des paupières, **caractérisé par le fait que** l'on utilise une composition solide selon la revendication 10, que l'on applique ladite composition non hydratée sur une surface humide telle que les lèvres, le visage, les cils, les sourcils ou les paupières.

15. Procédé de traitement cosmétique pour le soin et/ou le conditionnement et/ou l'hygiène de la peau, des cheveux, du cuir chevelu ou des muqueuses, **caractérisé par le fait que** l'on utilise une composition solide selon la revendication 11, que l'on mouille celle-ci en surface avec de l'eau ou une surface humide et que l'on applique ladite composition hydratée sur la peau, les cheveux, les cils, le cuir chevelu ou les muqueuses.

16. Procédé de traitement cosmétique pour le soin et/ou le conditionnement et/ou l'hygiène de la peau, des cheveux, du cuir chevelu ou des muqueuses, **caractérisé par le fait que** l'on utilise une composition solide selon la revendication 11, que l'on applique ladite composition non hydratée sur une surface humide telle que sur la peau, les cheveux, les cils, les sourcils, le cuir chevelu ou les muqueuses.

17. Procédé de coiffage et/ou de mise en forme des fibres kératiniques telles que les cheveux, **caractérisé par le fait que** l'on utilise une composition solide selon la revendication 12, que l'on mouille celle-ci en surface avec de l'eau ou une surface humide et que l'on applique ladite composition hydratée sur lesdites fibres kératiniques.

18. Procédé de coiffage et/ou de mise en forme des fibres kératiniques telles que les cheveux, **caractérisé par le fait que** l'on utilise une composition solide selon la revendication 12, que l'on applique ladite composition non hydratée sur une surface humide telle que lesdites fibres kératiniques.
